Europäisches Patentamt

European Patent Office

Office européen des brevets

(19)

(11) Publication number: **0 013 803**
**B1**

# (12) EUROPEAN PATENT SPECIFICATION

(45) Date of publication of patent specification: **10.11.82**

(21) Application number: **79302614.7**

(22) Date of filing: **16.11.79**

(51) Int. Cl.³: **A 01 N 59/14,**
**A 61 B 10/00** //(A01N59/14,
37/02)

(54) **A preservative for urine, a sample device for sampling urine and a method for preserving a urine sample.**

(30) Priority: **15.01.79 US 3237**

(43) Date of publication of application:
**06.08.80 Bulletin 80/16**

(45) Publication of the grant of the patent:
**10.11.82 Bulletin 82/45**

(84) Designated Contracting States:
**AT BE CH DE FR GB IT LU NL SE**

(56) References cited:
**FR - A - 326 334**
**US - A - 2 460 641**
**US - A - 4 116 066**

**CHEMICAL ABSTRACTS, vol. 54, no. 3, 10**
**February 1960, column 2497e**
*Columbus, Ohio, US*
**H. J. REHM et al.: "Action of antimicrobial**
**substances in combination"**

(73) Proprietor: **Becton, Dickinson and Company**
**Mack Centre Drive**
**Paramus New Jersey 07652 (US)**

(72) Inventor: **Mehl, Jack Judson**
**695 Reba Road**
**Landing, New Jersey (US)**

(74) Representative: **Ruffles, Graham Keith et al,**
**MARKS & CLERK 57-60 Lincoln's Inn Fields**
**London WC2A 3LS (GB)**

(56) References cited:
**CHEMICAL ABSTRACTS, vol. 7, no. 19, 10**
**October 1913, page 3530**
**Columbus, Ohio, US**
**G. E. EWE et al.: "Laboratory notes"**

**CHEMICAL ABSTRACTS, vol. 8, no. 8, 20 April**
**1914**
**Columbus, Ohio, US**
**W. M. DEHN et al.: "A comparison of various**
**preservatives of urine", Seiten 1440—1441**

Courier Press, Leamington Spa, England.

## A preservative for urine, a sample device for sampling urine and a method for preserving a urine sample

This invention relates to the preservation of urine specimens.

Bacterial quantitation of clean-voided urine specimens is employed to determine the presence of urinary tract infections in many cases. However, such specimens are often contaminated from exogenous sources, and in view of the fact that urine has the capability of supporting the proliferation of bacteria, multiplication of such contamination may occur, thereby resulting in false positives.

In order to prevent multiplication of contaminants, culturing or refrigeration of a urine specimen within two to four hours is recommended. In many cases, however, such culturing or refrigeration is not possible. There is thus a need for a preservative for urine specimens which is capable of preserving the urine to prevent multiplication of bacterial contaminants.

Powdered boric acid has been proposed as such a preservative but it has been found that powdered boric acid is toxic to some of the tested strains present in urine. In addition, powdered boric acid is not effective for preventing proliferation of some bacterial contaminant strains.

In accordance with the present invention, there is provided a liquid preservative for urine specimens. The preservative is comprised of boric acid and an alkali metal formate dissolved in a bacteriostatic liquid. The boric acid and alkali formate are dissolved in the bacteriostatic liquid in an amount effective to preserve a urine specimen.

We find that such a liquid preservative can be effective for preserving a urine specimen for a period of at least 24 hours, and in most cases, the preservative is capable of preserving a urine specimen for a period of 48 hours. The liquid preservatives in accordance with the present invention prevent undue proliferation of bacterial contaminants, and also are not unduly toxic to bacteria present in urine specimens.

The boric acid is generally present in the liquid preservative in an amount to provide a boric acid concentration in the preserved sample in the order of from 0.9 to 1.2 per cent (all percentages in this specification being by weight). The alkali metal formate is generally present in the preservative in an amount to provide a formate concentration in the urine sample of from 0.5 to 0.6 per cent. The alkali metal formate is typically either potassium or sodium formate.

The nature of the bacteriostatic liquid employed in the present preservative is not critical. Preferably the liquid is either water or glycerine, with glycerine being more preferred because boric acid is more soluble in glycerine, thereby permitting the use of smaller amounts of the total liquid preservative per unit of sample. In addition, it has been found that glycerine interacts with the boric acid and formate to provide for increased preservation; liquid perservatives in accordance with the invention which include glycerine as the bacteriostatic liquid have been found to be effective preservatives for a period of 48 hours.

The amount of boric acid and alkali metal formate can be varied within broad ranges. Usually the weight ratio of boric acid to alkali metal formate will be in the range 3:1 to 1:1, with 2.5:1 to 1.5:1 being most commonly adopted. To some extent the actual amounts employed will depend upon the bacteriostatic liquid which is used. For glycerine-based perservatives the boric acid will typically be present in amounts of 7 to 15%, with around 10% being preferred. For water-based preservatives, the boric acid will typically be present in amounts of 1.5 to 2.5%, with around 1% being preferred. Correspondingly the glycerine- and water-based preservatives will normally contain from 4 to 7% and from 1 to 1.5% of formate, respectively.

In accordance with a preferred aspect of the present invention, the liquid preservative is included in a sample container for a urine sample. More particularly, use can be made of an evacuated container, such as an evacuated tube, as described in US Patent No. 2.460.641.

In accordance with an especially preferred embodiment of the present invention there is provided an evacuated tube closed by a stopper and containing a preservative in accordance with the invention. The preservative is included in an amount sufficient to preserve the sample quantity which will be drawn into the tube upon piercing stopper. A urine sample can be transferred to tube as known in the art by piercing the stopper with a cannula. A sample cup for facilitating such introduction is disclosed in our US Patent No. 4116066 and the reader is referred to that Patent.

In this general manner, the evacuated container can include an amount of liquid perservative effective for preserving the predetermined amount of urine sample which will be drawn into the evacuated sample container. This facilitates handling of the sample, and ensures an effective amount of preservative.

The invention will be further described with respect to the following non-limiting example.

Example

Urine samples were preserved as follows:

A. *Prior Art — Boric Acid*

Urine sample containing 1% boric acid

B. *Prior Art — Boric Acid*

Urine sample containing 1.8% boric acid

C. *Present Invention*

Boric acid and sodium formate dissolved in glycerine and added to urine sample to provide 1% boric acid; 0.5% sodium formate; and 10% glycerine in urine. In this instance 5 ml of urine was added to 0.5 ml of preservative.

Each of the preservatives was tested for its effectiveness against bacteriuria. 0.1 ml of each preserved urine sample was diluted with 9.9 ml of sterile distilled water, and 0.1 ml of the diluted sample was transferred to culture plates for count determination after 0.24 and 48 hours.

The preservation ability was evaluated by determining whether there was an unacceptable rise or drop in the bacterial count, either of which indicates that the preservation is ineffective. The results were as follows:

1. *E. coli*
Composition A — Unacceptable drop in count after 24 hours.
Composition B — Unacceptable drop in count after 48 hours.
Composition C — Suitable.

2. *C. freundii*
Composition A — Unacceptable increase in count after 48 hours.
Composition B — Unacceptable drop in count after 48 hours.
Composition C — Suitable.

3. *S. pyogenes*
Composition A — Unacceptable drop after 24 hours.
Composition B — Unacceptable drop after 24 hours.
Composition C — Suitable.

4. *S. faecalis*
Composition A — Unacceptable rise after 24 hours.
Composition B — Unacceptable rise after 48 hours.
Composition C — Slight, acceptable rise after 48 hours.

5. *P. mirabilis*
Composition A — Suitable.
Composition B — Unacceptable drop after 48 hours.
Composition C — Suitable.

6. *P. morgani*
Composition A — Suitable.
Composition B — Unacceptable drop after 48 hours.
Composition C — Suitable.

7. *Pseudomonas spp.*
Composition A — Suitable.
Composition B — Unacceptable.
Composition C — Suitable.

All three compositions were found to be suitable as a preservative for *E. cloacae* and *K. pneumoniae*.

A composition in accordance with the invention using water as the bacteriostatic liquid (concentration in urine 1% boric acid; 0.5 sodium formate; 50% water) was found to be an effective preservative in the above tests over the 24 hour period, 2.5 ml of the preservative being mixed with 2.5 ml of urine. In some cases, after 48 hours, preservation was not effective.

The composition C of the present invention was also found to give comparable results to preservation of a urine sample by refrigeration over the 24 hours and 48 hour test periods.

The present invention is thus particularly advantageous in that, by preserving in accordance with the present invention, it is not necessary to effect refrigeration of a urine sample. Moreover, the present invention offers the advantage that urine preservation can be effected with small amounts of preservative, thereby eliminating the necessity of employing a dilution factor in the sample determination. Furthermore, by providing such a preservative in an evacuated container, handling is eased and a correct amount of preservative is added to a predetermined quantity of urine sample. Thus, for example, in accordance with a preferred embodiment, it is possible to employ 0.5 ml of a preservative comprised of boric acid and sodium formate dissolved in glycerine in a tube evacuated to draw 5.0 ml of a urine specimen. The preservative is preferably formulated to provide 1% boric acid and 0.5% sodium formate in the urine sample.

**Claims**

1. A liquid preservative for urine, the preservation comprising: boric acid and an alkali metal formate dissolved in a bacteriostatic liquid in an amount effective to preserve a urine sample.

2. A preservative as claimed in claim 1, wherein the liquid is water.

3. A preservative as claimed in claim 1, wherein the liquid is glycerine.

4. A preservative as claimed in claim 1, 2 or 3, wherein the formate is sodium formate.

5. A preservative as claimed in claim 4, wherein the preservation is formulated to provide from 0.9 to 1.2% of boric acid from 0.5 to 0.6% of sodium formate in a urine sample

6. A preservation as claimed in claim 5, wherein the preservation is formulated to provide about 1% boric acid, about 0.5% sodium formate and about 10% glycerine in a urine sample.

7. A sample device for sampling urine, the device comprising: an evacuated sample container for urine, and a liquid preservative for urine in said evacuated sample container, said liquid preservative being as claimed in any preceding claim.

8. A method for preserving a urine sample, the method comprising adding to the urine sample a liquid preservative as claimed in any of claims 1 to 6.

9. A method as claimed in claim 8, wherein the liquid is glycerine and 0.5 ml of preservative is added to 5.0 ml of urine.

## Patentansprüche

1. Flüssiges Konservierungsmittel für Urin, welches umfaßt: Borsäure und ein in einer bakteriostatischen Flüssigkeit aufgelöstes Alkalimetallformiat in einer Menge, die wirksam eine Urinprobe konserviert.

2. Konservierungsmittel wie im Anspruch 1 beansprucht, wobei die Flüssigkeit Wasser ist.

3. Konservierungsmittel wie im Anspruch 1 beansprucht, wobei die Flüssigkeit Glyzerin ist.

4. Konservierungsmittel wie in den Ansprüchen 1, 2 oder 3 beansprucht, wobei das Formiat Natriumformiat ist.

5. Konservierungsmittel wie im Anspruch 4 beansprucht, wobei die Zusammensetzung so gewählt ist, daß in einer Urinprobe 0,9 bis 1,2% Borsäure und 0,5 bis 0,6% Natriumformiat enthalten sind.

6. Konservierungsmittel wie im Anspruch 5 beansprucht, wobei die Zusammensetzung so gewählt ist, daß in einer Urinprobe etwa 1% Borsäure, etwa 0,5% Natriumformiat und etwa 10% Glyzerin enthalten sind.

7. Eine Probenvorrichtung für Urin, die umfaßt: eine evakuierten Probenbehälter für Urin und ein flüssiges Konservierungsmittel für Urin in dem evakuierten Probenbehälter, wobei das flüssige Konservierungsmitel so ausgebildet ist, wie es in den vorhergehenden Ansprüchen beansprucht ist.

8. Verfahren zur Konservierung einer Urinprobe, welches darin besteht, einer Urinprobe ein flüssiges Konservierungsmittel hinzuzugeben, wie es in einem der Ansprüche 1 bis 6 formuliert ist.

9. Verfahren wie im Anspruch 8 beansprucht, wobei die Flüssigkeit Glyzerin ist und 0,5 ml des Konservierungsmittels 5,0 ml Urin zugegeben werden.

## Revendications

1. Préservateur liquide pour urine, le préservateur comprenant de l'acide borique et un formate de métal alcalin dissous dans un liquide bactériostatique en une quantité efficace pour préserver un échantillon d'urine.

2. Préservateur tel que revendiqué dans la revendication 1, dans lequel le liquide est de l'eau.

3. Préservateur tel que revendiqué dans la revendication 1, dans lequel le liquide est de la glycérine.

4. Préservateur tel que revendiqué dans la revendication 1, 2 ou 3, dans lequel le formate est du formate de sodium.

5. Préservateur tel que revendiqué dans la revendication 4, dans lequel le préservateur est formulé de façon à présenter de 0,9 à 1,2% d'acide borique, de 0,5 à 0,6% de formate de sodium dans un échantillon d'urine.

6. Préservateur tel que revendiqué dans la revendication 5, dans lequel le préservateur est formulé de façon à présenter environ 1% d'acide borique, environ 0,5% de formate de sodium et environ 10% de glycérine dans un échantillon d'urine.

7. Dispositif pour recueillir l'urine, le dispositif comprenant: un récipient d'échantillon sous vide et un préservateur liquide pour urine dans ledit récipient d'échantillon sous vide, ledit préservateur liquide étant tel que revendiqué dans l'une quelconque des revendications précédentes.

8. Procédé pour préserver un échantillon d'urine, le procédé comprenant l'addition à un échantillon d'urine, d'un préservateur liquide selon l'une quelconque des revendications 1 à 6.

9. Procédé tel que revendiqué dans la revendication 8, dans lequel le liquide est de la glycérine et dans lequel 0,5 ml de préservateur est ajouté à 5,0 ml d'urine.